# EUROPÄISCHE PATENTSCHRIFT

(11) **EP 2 647 397 B1**
(45) Veröffentlichungstag und Bekanntmachung des Hinweises auf die Patenterteilung: **08.10.2014**
(21) Anmeldenummer: 13001560.5
(22) Anmeldetag: 26.03.2013
(51) Int. Cl.: A61M 1/16, A61M 1/28, A61K 45/06, A61J 1/20, A61K 9/10, A61K 31/7004, A61K 33/14

(54) **Saures Dialysekonzentrat**
Acidic concentrate for dialysis
Concentré acide pour dialyse

(30) Priorität: 07.04.2012 DE 102012007165
(43) Veröffentlichungstag der Anmeldung: 09.10.2013
(73) Patentinhaber: Völker, Manfred, 63825 Blankenbach (DE)
(72) Erfinder: Völker, Manfred, 63825 Blankenbach (DE)
(74) Vertreter: Flosdorff, Jürgen

(56) Entgegenhaltungen:
- WO-A1-03/059416
- DE-C1- 19 955 578
- DE-T2- 69 727 811
- DE-U1-202005 006 624
- US-A- 6 039 720
- US-B1- 6 689 393

## Beschreibung

### Hintergrund

Bei Abschwächung oder Versagen der Nierenfunktion im menschlichen Körper treten verschiedene Komplikationen auf. Es führt zur Konzentrationssteigerung von mehreren organischen Stoffen im Blut. Einige von diesen Substanzen sind Proteinmetaboliten, also Stoffe, die ihre Herkunft aus der Bearbeitung von Proteinen herleiten, z.B. Harnstoff und Kreatinin. Viele der Stoffe zeigen toxische Wirkung und sind unter dem allgemeinen Begriff Toxine bekannt. Toxine konnte man je nach Molekülgröße klassifizieren: kleine Moleküle unter 300 Dalton, wie Harnstoff (Molekulargewicht MG 60), Kreatinin (MG 113), mittelgroße Moleküle bis 12000 Dalton, wie Parathormon (MG 9424), beta-2-Mikroglobulin (MG 11818) und große Moleküle wie Myoglobine. Diese Stoffe dienen als Marker für akute Nierenprobleme. Eine verursachte urämische Vergiftung führt schließlich zum Tode, wenn diese Abfallprodukte nicht durch künstliche Methoden entfernt werden. Meist verbreitete therapeutische Methode ist die Hämodialyse, und danach die Peritonealdialyse.

Dialyseflüssigkeit (DF) oder Dialysat ist die Flüssigkeit, die während der Hämodialyse an einer Seite einer Membran innerhalb eines Dialysators benutzt wird, um das Blut des an Nierenversagen erkrankten Menschen zu reinigen. Durch kleine Membranporen werden die Toxine aus dem Blut in die DF und die Stoffe von der DF ins Blut diffundiert.

In der Regel wird die DF Lösung für die Hämodialyse am Ort der Behandlung in einer Hämodialysemaschine aus speziellen flüssigen Säure Konzentraten und einem pulvrigen Bikarbonat Konzentrat durch Proportionierung und Vermischen mit hochreinem behandeltem Wasser, also einem Säurekonzentrat und einem Basekonzentrat hergestellt. Dialyseflüssigkeit enthält die physiologisch wichtigsten anorganischen Kationen und Anionen: Natrium, Kalium, Calcium, Magnesium, Chloride, pH-Einstellungskomponenten oder Puffer (oft Bikarbonat, Acetat, Ascorbat, Zitrat oder Salzsaure) und ein osmotisches Agent (meistens Glucose oder Icodextrin). Ein Grund für die Aufteilung des Dialysekonzentrates in saures Konzentrat und Bikarbonat-Konzentrat liegt in der geringeren Löslichkeit von Magnesium und Calciumcarbonat in der Flüssigkeit, um die Ausfällung von Calcium/Magnesium als Karbonatsalze zu verhindern.

Das Säurekonzentrat enthält üblicherweise Natriumchlorid, Kaliumchlorid, Calciumchlorid, Magnesiumchlorid, Glucose und eine Säure. Das Bikarbonat-konzentrat besteht aus 1 mol/L Lösung von Natriumbikarbonat und wird oft aus pulverförmigem Natriumbikarbonat bereitgestellt.

Konzentratdosiersysteme zur Herstellung von DF umfassen Dosierungssysteme mit festgelegtem Volumen oder dynamische Systeme. Die ersteren benutzen festgelegte Volumina von Konzentrat und Wasser, um die fertige DF zu bilden. Dynamische Dosierungssysteme benutzen voreingestellte Flussmesser häufig in Kombination mit Proportionierungspumpen zur Einstellung der Menge an Säure- und Bikarbonatkonzentraten. Überwacht wird bei beiden Systemen das Mischungsverhältnis mittels Messung der Leitfähigkeitskonzentration.

Der Stand der Technik sieht eine Belieferung der Dialysezentren mit flüssigen, pulvrigen, pastösen Konzentraten vor und ist mit einem erheblichen Aufwand und chemischen Risiken verbunden.

Daher lag der Erfindung die Aufgabe zugrunde die Vorstufe eines Konzentrates (Superkonzentrat = SK) insbesondere für Säurekonzentrate darzustellen die den nachfolgend aufgeführten Zielvorstellungen entspricht und die die Nachteile des Standes der Technik weitgehend vermeidet.
1. Niedrige Fracht und Lagerkosten
   1.1 Lokale Herstellung der DF
   1.2 Zuordnung von SK als ungefährliches Gut
2. Chemische und physikalische Lagerstabilität und medizinische Verwendbarkeit der fertigen Dialyselösungen
3. Technisch einfach realisierbaren Herstellungsprozess von konzentrierter Mischung aus Rohkomponenten
   3.1 Einfache Qualitätskontrolle für einzelne Bestandteile
   3.2 Hohe Löslichkeitseffizienz beim Mischen
4. Technisch einfach realisierbaren Herstellungsprozess von flüssigem Dialysekonzentrat
5. Einhaltung von normativen Vorgaben für das fertige Konzentrat ohne in-house Kontrolle.

Um niedrige Fracht und Lagerkosten für das SK zu gewährleisten, muss die Dichte des SK möglichst hoch und das gesamte Volumen möglichst klein sein. Während der Lagerungszeit darf im Superkonzentrat auch keine chemische Reaktion verlaufen, die die Zusammensetzung des Konzentrats ändert. Unter anderem soll eine Verklumpung vermieden werden, damit eine homogene und vollständige Auflösung gewährleistet ist.

Das SK soll für die internationale Beförderung als ungefährliches Gut gelten. Nach Abschnitt 3.3.1 ADR, Sondervorschritt 597 sind Essigsäurelösungen mit bis zu 10% Säuregehalt kein Gefahrgut.

Unter dem dritten und vierten Punkt versteht man die Möglichkeit der Produktion mit möglichst einfachen Mitteln zur Herstellung von SK aus einzelnen Rohstoffen, wie auch eine Maschine für die Herstellung von flüssigem Dialysekonzentrat aus SK in den Dialysezentren.
Nach der Herstellung von Dialysekonzentrat aus SK soll das Dialysekonzentrat alle normativen medizinischen Vorgaben erfüllen.
Die vorliegende Erfindung sieht geeignete Mittel vor, um die aufgeführten Zielvorstellungen zu realisieren, dabei sind die genannten Begriffe wie Superkonzentrat (SK), Slurry, Basiskonzentrat im Zusammenhang mit den jeweiligen Textpassagen als Vorstufe für ein Hämodialysekonzentrat zu verstehen und sind sinngemäß verwandt.

### Patentübersicht

Das Thema Dialysekonzentratherstellung wurde in mehreren Patenten behandelt.

Im Patent DE 10 313 965 B3 ist eine Vorrichtung zur Herstellung von saurem Dialysekonzentrat beschrieben. Alle benötigen Stoffe mit Ausnahme von Wasser werden in einem Gefäß gemischt und später vor Ort mit Wasser zu Dialysekonzentrat verdünnt. Es gibt 3 Nachteile: relativ komplexer und teurer Mischgerätaufbau (z.B. Integration der Dichtemessung des flüssigen Konzentrats), Glucosepulver wird zusammen mit 100% Essigsäure in einem Gefäß gelagert (Probleme mit chemischer Stabilität, Punkt 2). Es ist auch sehr fraglich, ob das Gefäß mit flüssiger Säure als ungefährliches Gut beim Transport gelten kann (Punkt 1.2).

Das europäische Patent 0 605 395 B1 betrifft ein Verfahren und eine Vorrichtung zur Herstellung einer Dialyseflüssigkeit oder ein Konzentrat zur Herstellung von Dialyseflüssigkeit oder ein Dialysekonzentrat entweder aus Pulver in Form einer konzentrierten Flüssigkeit oder Pulver in getrockneter Form oder einer Aufschlämmung mit Zugabe von Wasser. Nachteilhaft ist hierbei die physikalische und chemische Stabilität der Rohkomponenten. Es wird nicht beachtet, dass im Fall einer Dialysekonzentratherstellung aus einer Aufschlämmung für einzelne Bestandteile (z.B. Glucose) ein anderer pH-Wert benötigt wird als für den Rest des Konzentrats, damit eine Langzeit -Stabilität gewährleistet ist.
Außerdem ist keine genaue Definition der Aufschlämmung vorgegeben ("in der Größenordnung von 40Gew.%").
Die Vorrichtung sieht auch vor, dass während der Herstellung Säure erst in flüssiger oder trockener Pulverform im Endprodukt zugegeben wird und nicht zusammen mit anderen Stoffen in der Eingangsmischung. Im Fall z.B. von Essigsäure, die flüchtig ist, kann dies zu Konzentrationsungenauigkeit führen. Zusätzlich ist die Verarbeitung konzentrierter Essigsäure problematisch.

Das Patent EP 0 456 928 B1 offenbart eine pastöse Zusammensetzung für die Dialysekonzentratherstellung, die aus 30-70% festen Elektrolytkomponenten (optional mit Glucose) und 70-30% Wasser besteht und ein Verfahren zur Herstellung der Zusammensetzung. Hier wird Natriumacetat und nicht Essigsäure als Acetatquelle genannt. Ein Nachteil ist die mangelhafte chemische Stabilität von Glucose. Nach diesem Verfahren werden alle Bestandteile des acetathaltigen Dialysekonzentrats zwar in Form einer Aufschlämmung vorbereitet, Glucose aber wird zusammen mit anderen Komponenten beigemischt. Das alles im Zusammenhang mit nicht optimalem pH-Wert führt innerhalb der Lagerzeit zur Glucosedegradation. Eine Zuordnung dieser Zusammensetzung als gefährliches Gut wird hier nicht erwähnt.

Das Patent DE 69 727 811 T2 beschreibt zur Verwendung in der Peritonealdialyse zwei separate Lösungen: eine glucosehaltigen Lösung mit Glucoseanteil von 20% bis 40 % bei einem pH-Wert von ungefähr 3,2 und eine Lösung mit verbleibenden Komponenten, die für die DF notwendig sind. Die Lösung mit Glucose wird sterilisiert und dann mit der zweiten Lösung gemischt. Das Verfahren weist eine verringerte Bildung von "Advanced Glycosylation Endproducts (AGEs)" auf. Das Patent beschreibt die Verwendung von Lösungen und nicht von Aufschlämmungen. Aus dem Patent wird nicht geklärt, wie die Lösung mit den unorganischen Salzen hergestellt wird - aus trockenen oder gemischten Rohrstoffen mit Wasser.

In einem früheren Patent DE 69 230 473 T2 wurde eine ähnliche Aufteilung zur Bereitung einer medizinischen DF beschrieben. Eine erste Packung enthält Glucose oder glucoseähnliche Verbindung in kleinerer Konzentration zusammen mit weiteren Substanzen, eine zweite Packung hat einen höheren Glucosegehalt als die erste. Der Gehalt der Glucose oder glucoseähnlichen Verbindungen in der zweiten Packung hat eine Konzentration von 40 Gew.-%.

Im Patent US 6 689 393 B1 werden drei Lösungen benutzt, um eine DF mit unterschiedlicher Glucosekonzentration vorzubereiten. DF-Lösungen mit unterschiedlicher Glucosekonzentration werden oft bei der Peritonealdialyse und auch bei sogenannter Profilierung (zeitliche Konzentrationsanpassung) verwendet. Die erste Lösung enthält Calcium und Elektrolytsalze, und optional Glucose in Konzentration weniger als 0,1 M(ca. 18g/L). Die zweite Lösung hat Glucose in anderer Konzentration als die erste Lösung und verbliebene Komponenten in gleichen Konzentrationen wie die erste Lösung. Der pH-Wert von beiden Lösungen ist mit einer Säure auf 3.7 eingestellt. Eine dritte Lösung ist ein alkalischer Puffer (mit Bikarbonat, Purivat, Laktat oder Alpha-ketoglutarationen). Das Mischen von allen drei Lösungen ermöglicht die Herstellung von Dialyseflüssigkeit mit unterschiedlichen Glucosekonzentrationen. Das Patent bezieht sich auf die Herstellung von Dialyseflüssigkeit nur aus echten Lösungen. Dies wird zu höheren Lager- und Transportkosten führen. Ein weiterer offensichtlicher Nachteil ist die relativ komplexe technische Entwicklung, Aufbau und Instandhaltung einer Mischanlage mit drei unterschiedlichen Lösungen.

Das ältere Patent DE 199 55 578 C1 beschreibt einen Mehrkammer-behälter für das Dialysekonzentrat mit drei Kompartimenten für den Einsatz in der Nierendialyse: einen für Glucosekonzentrat (über 10% Glucosegehalt, bevorzugt 20-30% und pH=3), und einen zweiten für Natrium, Calcium, Kaliumchlorid und eine Salzsäure anstatt Essigsäure. Hier wird nur Salzsäure als Säuerungsmittel genannt. Salzsäure ist eine starke Säure und kann den pH-Puffer stark beeinflussen. Salzsäure hat auch den Nachteil, nicht hitzesterilisierbar zu sein, da Chlorwasserstoff als Gas sich leicht aus der Lösung verflüchtigt. Das führt auch zu Problemen bei der Dosierung der Säure im Dialysekonzentrat. Salzsäure wirkt sehr korrosiv und wird weitere Schwierigkeiten bei der Produktion und Lagerung verursachen (Verstoß gegen Zielanforderung Nr. 4). Aus dem Patent wird nicht klar, aus welchen physikalischen Komponenten (Pulver, Lösungen, Mischungen) das Konzentrat hergestellt wird. Wenn für die Herstellung von Konzentraten reine Rohstoffe als Pulver oder hoch konzentrierte Lösungen benutzen werden, muss man mit höheren Transportkosten rechnen (Punkt 1). Auch ist die Zweck-bestimmung eines weiteren im wesentlichen leeren Fasskompartiment nicht erkennbar, was zu erhöhten Lagerkosten führt.

Im Patent DE 20 2005 006 624 U1 wird der vorgeschlagene Rohstoffe für ein Dialysekonzentrat in einer Verpackungseinheit verteilt. Ein Teil der Rohstoffe (K+, Ca2+, Mg2+ haltige Salze, konzentrierte Essigsäure) wird in flüssiger Form angesetzt, Kochsalz und Glucose kommt in trockener Form dazu. Hauptvorteil ist, dass es einfach zuzubereiten ist. Nachteil: durch Verwendung von trockenem Kochsalz und Glucose wird das Gesamtvolumen nicht optimiert, weil das Schüttvolumen von trockenen Substanzen höher als dasjenige von feuchten ist, mit daraus folgenden erhöhten Lagerkosten. Diese Verpackungs-einheit hat insgesamt vier separate Behälter, und das ergibt einen zusätzlichen technischen Aufwand beim Zusammenmischen (Punkt 4).

Eine Reihe von Patenten (z.B. US 6039720) beschreibt spezielle Container für sterile medizinische Lösungen. Der Container ist eine verbesserte Variante eines Fasses (Patent WO 93/09820) mit 2 oder 3 Abteilungen für Glucose/Glucose ähnliche Verbindung (Glucosegehalt mindestens 10%, pH-Wert um 3,5) und den Reststoffen. Als Einflussfaktoren auf eine Toxinbildung in der Glucose werden hoher pH-Wert, hohe Temperatur, Elektrolyte, Sauerstoff und Lagerzeit genannt. Beide Patente beschäftigen sich mit Dialysekonzentraten und nicht mit einer Zusammensetzung für die Herstellung von SK; d.h. in der Ausführung wird Dialysekonzentrat getrennt (Glucose und der Rest) gelagert und nicht als Slurry.

Die Patente DE 19 931 077 B4 und DE 10 100 462 B4 offenbaren Verfahren zur Herstellung von saurem Dialysekonzentrat und Behälter zur Durchführung des Verfahrens. Die Aufschlämmung liegt in einem Behältnis vor und besteht aus 72-90% Rohstoffanteil und 28-10% Wasser. Damit wird das Volumen gegenüber herkömmlichen Sauren Dialysekonzentraten minimiert. Als zusätzlicher Vorteil wird die notwendige Homogenität des Konzentrats gewährleistet. Zum Nachteil zählt, dass Glucose sich zusammen mit allen Komponenten in einem sehr sauren Medium mit einem pH-Wert von ca. 1,4 befindet, wobei dies nicht optimal ist, um Glucosezerfallsprodukte zu vermeiden (geringere Lagerzeit). Zum weiteren Nachteil in dieser Konzentratformulierung zählt, dass es nicht möglich ist, zuverlässige Proben von Glucose zu entnehmen, um die Qualität und Zusammensetzung zu überprüfen.

### Patente über Doppelkammerbeutel

Die Patente EP 1 354 607 B1 und DE 10 217 356 beschreiben eine Rezeptur für die Peritonealdialyse und einen Doppelkammerbeutel für die Dialyse. Die erste Einzellösung besteht aus einem Osmotikum (Glucosepolymer und/oder Glucosepolymerderivat) bei einem pH-Wert im Bereich zwischen 3,5 bis 5,0, vorzugsweise 4,2, sowie Calcium, Natrium, Magnesium und Chlorid-Ionen.
Die zweite Lösung ist ein Puffer. Der Doppelkammerbeutel ist ein Kunststoffbeutel mit zwei Kammern, die benachbart angeordnet sind. Eine Schweißnaht trennt die Kammern und öffnet sich bei einem Druck auf eine der Kammern. Unterschiede sind
- Gebrauchsfertige Lösungen, kein Konzentrat
- Glucosepolymer, keine Glucose
- Glucosepolymer mit pH 4,2 in der ersten Kammer, Puffer in der zweiten Kammer
- kein Acetat, Salzsäure wird bevorzugt
- benachbarte Kammeranordnung
- unklar ist die Anschlusszahl.

Das Patent DE 3 833 036 C2 beschreibt eine Doppelkammerampulle mit entfernbaren Verschlüssen. Nach der Entfernung der Verschlüsse steckt man zwei Ampullen ineinander, und beide Flüssigkeiten werden vermischt. Das Patent bezieht sich auf kleine Volumina und ist hier nicht relevant.

Die Patente DE 69 608 493 T2 und DE 696 062 10 T3 beschreiben einen flexiblen und mit zwei Kammern versehenen Behälter. Das System dient zu Aufbewahrung eines Feststoffes und einer Flüssigkeit. Beide Kammern sind mit mehrschichtigen Folien konstruiert, um Wasserdampf-, Sauerstoff- und Lichtzugang zu verhindern. Die Kammern sind mit einer durchbrech- bzw. zerreißbaren schwachen Dichtung versehen. Unterschied zum vorliegenden Verpackungssystem:
- das System ist nur für einen Feststoff und eine Flüssigkeit vorgesehen,
- die Kammern sind nebeneinander verbunden,
- das System ist schlecht skalierbar d.h. in unterschiedlich großen Verpackungseinheiten zu liefern.

Das Gebrauchsmuster DE 880 376 6 offenbart eine Spritze mit einer Kammerscheidewand. Durch Betätigung einer Stange werden Öffnungen zwischen zwei Kammern geöffnet. Durch Schieben der Scheidewand kann die entstandene Mischung aus der Spritze ausgedrückt werden. Wichtige Merkmale:
- es handelt sich um eine Applikation außerhalb der Hämodialyse
- ist nicht automatisierbar
- die Spritze ist nicht geeignet, um große Stoffvolumina zu mischen.

Eine andere Zweikammerampulle beschreibt das Patent DE 103 04 500 A1. Diese Konstruktion hat dieselben Nachteile wie das vorherige Gebrauchsmuster.

Der vorliegenden Erfindung liegt die Aufgabe zugrunde, eine Vorstufe eines sauren Dialysekonzentrats anzugeben, die lange lagerbar ist, niedrige Fracht- und Lagerkosten mit sich bringt und aus der auf einfache Weise flüssiges Dialysekonzentrat herstellbar ist.

Diese Aufgabe wird erfindungsgemäß durch die Merkmale des Patentanspruchs 1 gelöst.

Vorteilhafte Ausgestaltungen der Erfindung sind in den Unteransprüchen gekennzeichnet.

Um obengenannte Zielvorstellungen zu erfüllen, werden folgenden Mischungen für das SK vorgeschlagen.
1. Superkonzentrat (SK) wird auf 2 Teile dividiert: In einem Teil wird Glucose, vorzugsweise Glucosemonohydrat mit Wasser im Mischverhältnis von bevorzugt 2,4:1 (70,6 Gew.-%:29,4% Gew.-%):in Form einer Aufschlämmung zusammengemischt. Eine geringe Menge von Säuremittel (Essigsäure, Zitronensäure o.a. physiologisch einsetzbare Säuren) wird benutzt, um den pH-Wert im Bereich 2-3,2 (bevorzugt pH=3.0) einzustellen.
2. Der zweite Teil des SK besteht bevorzugt aus allen für das SK benötigten Stoffen, außer Glucose: nämlich Natriumchlorid, Kaliumchlorid, Calcium-chlorid, Magnesiumchlorid oder jeweilige Hydrate und den Rest der Essigsäure. Dieser Teil wird mit Wasser auch bis zum Aufschlämmungs-zustand verdünnt. Die zugegebene Wassermenge soll gerade so groß sein, um die Essigsäurekonzentration umgerechnet auf den gesamten Wasseranteil (Wasser aus Salzhydratreste plus zugegebenes Wasser) unter 10% in diesem Teil des SK zu halten.
3. Chemische Zusammensetzung:
   Der Saure Teil des SK (Saure Basis) enthält bevorzugt Natriumchlorid, Kaliumchlorid, Calciumchloriddihydrat, Magnesiumchloridhexahydrat und Essigsäure.
      Der Glukose Teil des SK (Glucose Basis) enthält Glucose und vorzugsweise zusätzlich KCI, und/oder MgCl₂ und/oder CaCl₂ und geringe Menge an Essigsäure. Die Salzmengen sind so ausgewählt, dass sie in einer gesättigten Glucoselösung bei einer Temperatur von ca. 5°C komplett auf zu lösen sind. Die sogenannte Glucose Basis ist mit Essigsäure auf pH=3,0 eingestellt.
      Beide Teile vom Konzentrat haben eine Essigsäurekonzentration von weniger als 10% in der flüssigen Phase.
      Das Verhältnis Feststoffe zu Flüssigkeit im Teil Saure Basis ist bevorzugt 72 Gew.-%, im Glucose Basis Teil bevorzugt 64 Gew.-% und im gesamten Slurry, d.h. Feststoffe + Wasser +Säure) ca. 70 Gew.-%.
      Das Gewichtsverhältnis von Teil 1 zu Teil 2 ist 4,5 bis 5,1:1. Das Volumenverhältnis von Teil 1:Teil 2 ist 3,2 bis 3,6:1.
Sowohl im Teil 1 als auch im Teil 2 des SK können die jeweiligen Anteile an Kaliumchlorid, Calciumchlorid, Magnesiumchlorid oder die jeweilige Hydrate und die Säure bedarfsgerecht, bereits mit Wasser vorverdünnt und vorgelöst der pulvrigen Glukose, bzw. dem pulvrigen Natriumchlorid während des Produktions-prozesses zugeführt werden.

Die erfindungsgemäße Zusammensetzung für 100 l Konzentrat und auch für 1 Patienten-Behandlung ist in den Tabellen für 1:44 und 1:34 Verdünnung zum Zwecke einer anwendungsgerechten Applikation beispielhaft zusammenfasst.

Die Erfindung sieht eine oben auch als Superkonzentrat (SK) oder auch als Slurry bezeichnete Vorstufe eines Sauren Dialysekonzentrats vor, die als zwei räumlich getrennte Teilkonzentrate bereit gestellt und zum Anwendungsort transportiert wird. Die Teilkonzentrate sind über einen sehr langen Zeitraum lagerbar, ohne dass die Glukose chemisch verändert bzw. abgebaut wird. Das Transport-volumen der Teilkonzentrate ist verhältnismäßig gering, da beide Teilkonzentrate in Form einer Aufschlämmung mit nur teilweise gelösten Bestandteilen vorliegen, wobei diese Aufschlämmungen ein verringertes Volumen als trockene Feststoffe haben. Am Ort der Anwendung werden die Teilkonzentrate des Superkonzentrats zu einem sauren Dialysekonzentrat verdünnt. Bedingt durch die Aufschlämmung wird eine hohe Löslichkeitseffizienz im Vergleich zu trockenen Konzentraten während des Mischvorganges erzielt. In den Dialysemaschinen wird das saure Dialysekonzentrat zu Dialysierflüssigkeit zubereitet.

### 2-Teile Zusammensetzung 1:44 für 100 L saures Konzentrat, ca. 30L(45,843kg) SLURRY mit 74,970L Aqua purificata

| **Sorte** | **NaCl , kg** | **KCl, kg** | **CaCl2x 2H2O,** | **MgCl2x 6H2O, kg** | **CH3CO OH, kg** | **Wasser , L** | **Glucose xH2O, kg** | **Wasser ,L** | **KCl, kg** | **MgCl2x 6H2O, kg** | **CH3CO OH, kg** |
|---|---|---|---|---|---|---|---|---|---|---|---|
| | **Saure Vorstufe** | | | | | | **Glucose Vorstufe** | | | | |
| Konzentrat 1 | 27,088 | 0,062 | 0,827 | 0,405 | 0,805 | 8,054 | 4,950 | 2,057 | 0,609 | 0,053 | 0,006 |
| Konzentrat 2 | 27,088 | 0,062 | 0,992 | 0,405 | 0,805 | 8,016 | 4,950 | 2,057 | 0,609 | 0,053 | 0,006 |
| Konzentrat 3 | 27,088 | 0,397 | 0,992 | 0,405 | 0,805 | 7,862 | 4,950 | 2,057 | 0,609 | 0,053 | 0,006 |
| Konzentrat 4 | 27,088 | 0,397 | 0,827 | 0,405 | 0,805 | 7,901 | 4,950 | 2,057 | 0,609 | 0,053 | 0,006 |
| Konzentrat 5 | 27,088 | 0,733 | 0,992 | 0,405 | 0,805 | 7,720 | 4,950 | 2,057 | 0,609 | 0,053 | 0,006 |
| Konzentrat 6 | 27,088 | 0,733 | 0,827 | 0,405 | 0,805 | 7,746 | 4,950 | 2,057 | 0,609 | 0,053 | 0,006 |
| Konzentrat 7 | 27,614 | 0,000 | 0,827 | 0,405 | 1,075 | 7,938 | 4,950 | 2,057 | 0,335 | 0,053 | 0,006 |
| Konzentrat 8 | 27,614 | 0,062 | 0,827 | 0,405 | 1,075 | 7,810 | 4,950 | 2,057 | 0,609 | 0,053 | 0,006 |
| Konzentrat 9 | 27,614 | 0,397 | 0,827 | 0,405 | 1,075 | 7,682 | 4,950 | 2,057 | 0,609 | 0,053 | 0,006 |
| Konzentrat 10 | 27,614 | 0,733 | 0,827 | 0,405 | 1,075 | 7,514 | 4,950 | 2,057 | 0,609 | 0,053 | 0,006 |

### 2-Teile Zusammensetzung 1:44 für 2,667 L saures Konzentrat, ca. 1,23L(0,87kg) SLURRY mit 1,999L Aqua purificata 1 Patient benötigt ca. 1,23 kg Slurry bei 5 stündiger Dialyse (Dialysatfluß ca. 0,4L/Min)

| **Sorte** | **NaCl, kg** | **KCl, kg** | **CaCl2 x2H2O, kg** | **MgCl2 x6H2O, kg** | **CH3CO OH, kg** | **Wasser, L** | **Glucose xH2O,kg** | **Wasser, L** | **KCl, kg** | **MgCl2 x6H2O, kg** | **CH3CO OH, kg** |
|---|---|---|---|---|---|---|---|---|---|---|---|
| | **Saure Vorstufe** | | | | | | **Glucose Vorstufe** | | | | |
| Konzentrat 1 | 0,722 | 0,002 | 0,022 | 0,011 | 0,021 | 0,215 | 0,132 | 0,055 | 0,016 | 0,001 | 0,00017 |
| Konzentrat 2 | 0,722 | 0,002 | 0,026 | 0,011 | 0,021 | 0,214 | 0,132 | 0,055 | 0,016 | 0,001 | 0,00017 |
| Konzentrat 3 | 0,722 | 0,011 | 0,026 | 0,011 | 0,021 | 0,210 | 0,132 | 0,055 | 0,016 | 0,001 | 0,00017 |
| Konzentrat 4 | 0,722 | 0,011 | 0,022 | 0,011 | 0,021 | 0,211 | 0,132 | 0,055 | 0,016 | 0,001 | 0,00017 |
| Konzentrat 5 | 0,722 | 0,020 | 0,026 | 0,011 | 0,021 | 0,206 | 0,132 | 0,055 | 0,016 | 0,001 | 0,00017 |
| Konzentrat 6 | 0,722 | 0,020 | 0,022 | 0,011 | 0,021 | 0,207 | 0,132 | 0,055 | 0,016 | 0,001 | 0,00017 |
| Konzentrat 7 | 0,736 | 0,000 | 0,022 | 0,011 | 0,029 | 0,212 | 0,132 | 0,055 | 0,009 | 0,001 | 0,00017 |
| Konzentrat 8 | 0,736 | 0,002 | 0,022 | 0,011 | 0,029 | 0,208 | 0,132 | 0,055 | 0,016 | 0,001 | 0,00017 |
| Konzentrat 9 | 0,736 | 0,011 | 0,022 | 0,011 | 0,029 | 0,205 | 0,132 | 0,055 | 0,016 | 0,001 | 0,00017 |
| Konzentrat 10 | 0,736 | 0,020 | 0,022 | 0,011 | 0,029 | 0,200 | 0,132 | 0,055 | 0,016 | 0,001 | 0,00017 |

### 2-Teile Zusammensetzung 1:34 für 100 L saures Konzentrat, ca. 23L(33,623kg) SLURRY (Feststoffe + Wasser + Säure)

| **Sorte** | **NaCl, kg** | **KCl, kg** | **CaCl2 x2H2O, kg** | **MgCl2 x6H2O, kg** | **CH3COOH, kg** | **Wasser, L** | **Glucose xH2O, kg** | **Wasser, L** | **KCl, kg** | **MgCl2 x6H2O, kg** | **CH3COOH, kg** |
|---|---|---|---|---|---|---|---|---|---|---|---|
| | **Saure Vorstufe** | | | | | | **Glucose Vorstufe** | | | | |
| Konzentrat 1 | 0,722 | 0,002 | 0,026 | 0,011 | 0,021 | 0,155 | 0,132 | 0,055 | ^{.}0,016 | 0,0014 | 0,00017 |
| Konzentrat 2 | 0,722 | 0,002 | 0,022 | 0,011 | 0,021 | 0,156 | 0,132 | 0,055 | 0,016 | 0,0014 | 0,00017 |
| Konzentrat 3 | 0,722 | 0,011 | 0,026 | 0,011 | 0,021 | 0,150 | 0,132 | 0,055 | 0,016 | 0,0014 | 0,00017 |
| Konzentrat 4 | 0,722 | 0,011 | 0,022 | 0,011 | 0,021 | 0,152 | 0,132 | 0,055 | 0,016 | 0,0014 | 0,00017 |
| Konzentrat 5 | 0,722 | 0,020 | 0,026 | 0,011 | 0,021 | 0,148 | 0,132 | 0,055 | 0,016 | 0,0014 | 0,00017 |
| Konzentrat 6 | 0,722 | 0,020 | 0,022 | 0,011 | 0,021 | 0,148 | 0,132 | 0,055 | 0,016 | 0,0014 | 0,00017 |

### 2-Teil Zusammensetzung 1:34 für 3,429 L saures Konzentrat, ca.0,79L(1,153kg) SLURRY 1 Patient braucht ca. 1,153 kg Slurry (1:34) bei 5 stündiger Dialyse (Dialysatfluß ca. 0,4L/Min)

| **Sorte** | **NaCl, kg** | **KCl, kg** | **CaCl2 x2H2O, kg** | **MgCl2 x6H2O, kg** | **CH3CO OH, kg** | **Wasser, L** | **Gluc. xH2O, kg** | **H₂O, L** | **KCI, kg** | **MgCl2 x6H2O, kg** | **CH3COOH, kg** |
|---|---|---|---|---|---|---|---|---|---|---|---|
| | **Saure Vorstufe** | | | | | | **Glucose Vorstufe** | | | | |
| Konzentrat 1 | 21,069 | 0,048 | 0,772 | 0,315 | 0,626 | 4,533 | 3,850 | 1,600 | 0,474 | 0.041 | 0.005 |
| Konzentrat 2 | 21,069 | 0,048 | 0,643 | 0,315 | 0,626 | 4,563 | 3,850 | 1,600 | 0,474 | 0,041 | 0,005 |
| Konzentrat 3 | 21,069 | 0,309 | 0,772 | 0,315 | 0,626 | 4,363 | 3,850 | 1,600 | 0,474 | 0,041 | 0,005 |
| Konzentrat 4 | 21,069 | 0,309 | 0,643 | 0,315 | 0,626 | 4,442 | 3,850 | 1,600 | 0,474 | 0,041 | 0,005 |
| Konzentrat 5 | 21,069 | 0,570 | 0,772 | 0,315 | 0,626 | 4,302 | 3,850 | 1,600 | 0,474 | 0,041 | 0,005 |
| Konzentrat 6 | 21,069 | 0,570 | 0,643 | 0,315 | 0,626 | 4,322 | 3,850 | 1,600 | 0,474 | 0,041 | 0,005 |

## Patentansprüche

1. Vorstufe eines sauren Dialysekonzentrats,
wobei aus den Inhaltsstoffen des sauren Dialysekonzentrats zwei getrennt bereit gestellte Teilkonzentrate gebildet sind, wobei nur das eine Teilkonzentrat Glukose enthält, während das andere Teilkonzentrat keine Glukose aufweist,
wobei das eine Teilkonzentrat neben Glukose Wasser, ein Säuremittel und KCI und/oder MgCl₂ und/oder CaCl₂ enthält und die Salzmengen so ausgewählt sind, dass sie in einer gesättigten Glukoselösung bei einer Temperatur von ca. 5°C komplett lösbar sind, wobei Bestandteile der Glukose im ungelösten Zustand verbleiben, so dass das eine Teilkonzentrat in einem Aufschlämmungszustand vorliegt, wobei ferner das eine Teilkonzentrat auf einen pH-Wert von 2 bis 3.2, vorzugsweise 3.0 eingestellt ist,
und wobei das andere Teilkonzentrat Natriumchlorid und/oder Kaliumchlorid und/oder Calciumchlorid und/oder Magnesiumchlorid oder jeweilige Hydrate, Wasser und ein Säuremittel enthält und ebenfalls in einem Aufschlämmungszustand vorliegt, in dem Teile des Natriumchlorids im ungelösten Zustand verbleiben und die anderen Bestandteile gelöst hinzugefügt sind.

2. Vorstufe eines sauren Dialysekonzentrats nach Anspruch 1,
**dadurch gekennzeichnet,**
**dass** das eine Teilkonzentrat Glukosemonohydrat aufweist.

3. Vorstufe eines sauren Dialysekonzentrats nach Anspruch 1 oder 2,
**dadurch gekennzeichnet,**
**dass** das eine und das andere Teilkonzentrat als Säuremittel Essigsäure enthalten.

4. Vorstufe eines sauren Dialysekonzentrats nach Anspruch 3,
wobei beide Teilkonzentrate Essigsäurekonzentrationen in flüssiger Phase unter 10 Gew.-% aufweisen.

5. Vorstufe eines sauren Dialysekonzentrats nach den Ansprüchen 1 bis 4,
**dadurch gekennzeichnet,**
**dass** das Verhältnis der Feststoffe zu Wasser in dem einen Teilkonzentrat 60 bis 64 Gew.-% beträgt.

6. Vorstufe eines sauren Dialysekonzentrats nach den Ansprüchen 1 bis 5,
**dadurch gekennzeichnet,**
**dass** das Verhältnis Feststoff zu Wasser in dem anderen Teilkonzentrat 71 bis 82 Gew.-% beträgt.

7. Vorstufe eines sauren Dialysekonzentrats nach den Ansprüchen 1 bis 6 ,
**dadurch gekennzeichnet,**
**dass** die beiden Teilkonzentrate in einem Behälter mit zwei getrennten Kammern untergebracht sind.

## Claims

1. A precursor of an acid dialysis concentrate, wherein two separately prepared component concentrates are formed from the ingredients of the acid dialysis concentrate, wherein only the one component concentrate contains glucose whilst the other component concentrate includes no glucose, wherein, in addition to glucose, the one component concentrate contains water, an acid agent and KCI and/or MgCl₂ and/or CaCl₂ and the salt quantities are so selected that they are completely soluble in a saturated glucose solution at a temperature of ca. 5°c, wherein components of the glucose remain in the undissolved state, so that the one component concentrate is present in a form of a suspension, wherein further the one component concentrate is set to a pH value of 2 to 3.2, preferably 3.0, and wherein the other component concentrate includes sodium chloride and/or potassium chloride and/or calcium chloride and/or magnesium chloride or their respective hydrate, water and an acid agent and is also present in the form of a suspension, in which portions of the sodium chloride remain in the undissolved state and the other components are added in dissolved form.

2. A precursor of an acid dialysis concentrate as claimed in claim 1, **characterised in that** the one component concentrate includes glucose monohydrate.

3. A precursor of an acid dialysis concentrate as claimed in claim 1 or 2, **characterised in that** the one and the other component concentrates contain acetic acid as the acid agent.

4. A precursor of an acid dialysis concentrate as claimed in claim 3, wherein both component concentrates include acetic acid concentrations in the liquid phase of less than 10 wt.%.

5. A precursor of an acid dialysis concentrate as claimed in claims 1 to 4, **characterised in that** the ratio of the solid materials to water in the one component concentrate is 60 to 64 wt. %.

6. A precursor of an acid dialysis concentrate as claimed in claims 1 to 5, **characterised in that** the ratio of solid material to water in the other component concentrate is 71 to 82 wt. %.

7. A precursor of an acid dialysis concentrate as claimed in claims 1 to 6, **characterised in that** the two component concentrates are accommodated in a container with two separate chambers.

## Revendications

1. Précurseur d'un concentré de dialyse acide,
dans lequel à partir des composants du concentré de dialyse acide, deux concentrés partiels mis à disposition séparément sont formés, dans lequel seul le premier concentré partiel contient du glucose alors que l'autre concentré partiel ne contient pas de glucose,
dans lequel le premier concentré partiel contient, outre le glucose, de l'eau, un agent acide et du KCl et/ou MgCl₂ et/ou CaCl₂ et les quantités de sel sont choisies de telle sorte qu'elles sont dissoutes complètement dans une solution de glucose saturée à une température d'environ 5 °C, dans lequel des composants du glucose restent à l'état non dissous de sorte que le premier concentré partiel se trouve à l'état de suspension, dans lequel, en outre, le premier concentré partiel est ajusté à un pH de 2 à 3,2, de préférence de 3,0,
et dans lequel l'autre concentré partiel contient du chlorure de sodium et/ou du chlorure de potassium et/ou du chlorure de calcium et/ou du chlorure de magnésium ou des hydrates respectifs, de l'eau et un agent acide et se présente également à l'état de suspension, dans lequel des parties du chlorure de sodium restent à l'état non dissous et les autres composants sont ajoutés à l'état dissous.

2. Précurseur d'un concentré de dialyse acide selon la revendication 1,
**caractérisé en ce que**
le premier concentré partiel présente du glucose monohydraté.

3. Précurseur d'un concentré de dialyse acide selon la revendication 1 ou 2,
**caractérisé en ce que**
le premier concentré partiel et l'autre concentré partiel contiennent de l'acide acétique comme agent acide.

4. Précurseur d'un concentré de dialyse acide selon la revendication 3,
dans lequel les deux concentrés partiels présentent des concentrations d'acide acétique en phase liquide de moins de 10 % en poids.

5. Précurseur d'un concentré de dialyse acide selon les revendications 1 à 4,
**caractérisé en ce que**
le rapport des solides à l'eau dans le premier concentré partiel est de 60 à 64 % en poids.

6. Précurseur d'un concentré de dialyse acide selon les revendications 1 à 5,
**caractérisé en ce que**
le rapport des solides à l'eau dans l'autre concentré partiel est de 71 à 82 % en poids.

7. Précurseur d'un concentré de dialyse acide selon les revendications 1 à 6,
**caractérisé en ce que**
les deux concentrés partiels sont intégrés dans un contenant comportant deux chambres séparées.
